# EUROPEAN PATENT APPLICATION

(11) **EP 3 157 236 A1**
(43) Date of publication of application: **19.04.2017**
(21) Application number: 14894447.3
(22) Date of filing: 21.08.2014
(51) Int. Cl.: H04M 3/50, G10L 15/26, G06F 17/30

(54) **METHOD AND DEVICE FOR QUICKLY ACCESSING IVR MENU**

(30) Priority: 11.06.2014 CN 201410256760
(71) Applicant: ZTE Corporation, Shenzhen, Guangdong 518057 (CN)
(72) Inventor: ZHU, Shizheng, Shenzhen Guangdong 518057 (CN)
(74) Representative: Potter Clarkson LLP
(86) International application number: PCT/CN2014/084951
(87) International publication number: WO 2015/188454

(57) **Abstract**

Provided are a method and device for quickly accessing an IVR menu, the method comprising: matching with an IVR menu based on a user voice, and providing a user with a menu item matching the user voice. The device comprises a matching module and a processing module. The present invention enables a user to quickly access a desired menu item in the IVR menu directly via natural language interaction, thus reducing the waiting time of the user, and improving IVR use experience. Preferably, with additional IVR menu flattening, and when the user voice input is not quite accurate, the present invention can actively guide the user step by step according to the previous input voices of users, allowing the user to quickly access the desired menu.

## Description

### TECHNICAL FIELD

The present disclosure relates to the technical field of IVR (Interactive Voice Response) system, and particularly, to a method and device for quickly accessing an IVR menu.

### BACKGROUND

The IVR system is an important portal for a call center to implement a man-machine interaction. In the conventional IVR system, an interaction manner between a user and the system is mainly based on a keyboard of a telephone. However, as continuous increase of complexity of business logics and menus, for accessing a corresponding IVR menu item, the user often needs to press keys for many times or wait a menu prompt voice for a long time, thereby the interaction between the user and the IVR system is greatly restricted, which becomes a most dissatisfactory place during the usage experience of the user. In addition, there is another case that when the user mistakenly presses a key after accessing a certain level of IVR menu, the main menu may be returned to; at this time, it is inevitably to waste time of the user for re-selecting the menu item from top to bottom, and the efficiency of using the IVR system by the user is greatly affected.

### SUMMARY

The technical problem to be solved by embodiments of the present disclosure is to provide a method and device for quickly accessing an IVR menu, which can overcome the deficiency brought out by accessing an IVR menu item via pressing keys in the related art.

The technical solution adopted by the embodiments of the present disclosure is a method for quickly accessing an IVR menu, including:
performing a matching in the IVR menu based on voice of a user, and providing a menu item matched with the voice of the user to the user.

In an embodiment, the performing the matching in the IVR menu based on the voice of the user includes:
establishing an index of menu items based on a text corresponding to the IVR menu; and
matching a text corresponding to the voice of the user with the index of the menu items so as to find out the menu item matched with the voice of the user.

In an embodiment, the IVR menu is an IVR menu which has undergone a flattening process, and
the flattening process includes: with respect to menu items in two adjacent levels in the IVR menu, replacing the menu item in a current level by the menu item in a corresponding next level.

In an embodiment, a procedure of acquiring the text corresponding to the voice of the user includes:
after prompting the user an available function of the menu item via voice, recording the voice of the user and converting it into a user voice text, and then preprocessing the user voice text, the preprocessing including: a process of removing a stopword, and filtering out a character not encoded according to GBK (Chinese Internal Code Specification) and a non-word character.

In an embodiment, the procedure of acquiring the text corresponding to the voice of the user further includes:
splitting the preprocessed user voice text.

In an embodiment, the providing the menu item matched with the voice of the user to the user includes:
A1: determining whether a number of the menu item matched with the voice of the user is 1; if yes, providing a business logic functionality of the menu item to the user; otherwise, performing step A2; and
A2: performing a voice guidance for the user based on the user voice text, so as to prompt the user to input more accurate voice and perform the matching again.

The embodiments of the present disclosure also provides a device for quickly accessing an IVR menu, including:
a matching module configured to perform a matching in the IVR menu based on voice of a user; and
a processing module configured to provide a menu item matched with the voice of the user to the user.

In an embodiment, the matching module includes:
an index establishing module configured to establish an index of menu items based on a text corresponding to the IVR menu; and
a text matching module configured to match a text corresponding to the voice of the user with the index of the menu items so as to find out the menu item matched with the voice of the user.

In an embodiment, the matching module includes:
a menu processing module configured to perform a flattening process on the IVR menu and then provide it to the matching module, and
the flattening process includes: with respect to menu items in two adjacent levels in the IVR menu, replacing the menu item in a current level by the menu item in a corresponding next level.

The present disclosure also provides an IVR system, including:
a recording device configured to record voice of a user and convert it into a user voice text; and
a processor configured to establish an index of menu items based on a text corresponding to an IVR menu, match the user voice text with the index of the menu items so as to find out the menu item matched with the voice of the user, and provide business logic functionality of the menu item to the user.

By adopting the above technical solutions, the embodiments of the present disclosure at least have the following advantages.

Through the method and device for quickly accessing the IVR menu provided by the embodiments of the present disclosure, the user may directly interact with the IVR menu via natural language so as to quickly reach the desired menu item in the IVR menu, which saves the waiting time of the user and improves the usage experience of the IVR. Preferably, the flattening process is performed on the IVR menu to reduce the levels of the menu. When the voice inputted by the user is not accurate, the user can be actively guided level by level according to the voice inputted by the user previously, such that the user can quickly go into the desired menu.

### BRIEF DESCRIPTION OF THE DRAWINGS

Fig. 1 is a flow chart of a method for quickly accessing an IVR menu according to a first embodiment of the present disclosure;
Fig. 2 is a schematic diagram of constituent structure of a device for quickly accessing an IVR menu according to a second embodiment of the present disclosure;
Fig. 3 is a schematic diagram of constituent structure of an IVR system according to a third embodiment of the present disclosure;
Fig. 4 is a flow chart executed by a semantic analysis module according to the third embodiment of the present disclosure; and
Fig. 5 is a flow chart of quickly accessing the IVR menu according to the third embodiment of the present disclosure.

### DETAILED DESCRIPTION

To further explain the technical means adopted by the present disclosure for achieving predefined purposes and effects, detailed description will be given hereinafter in combination with drawings and preferred embodiments.

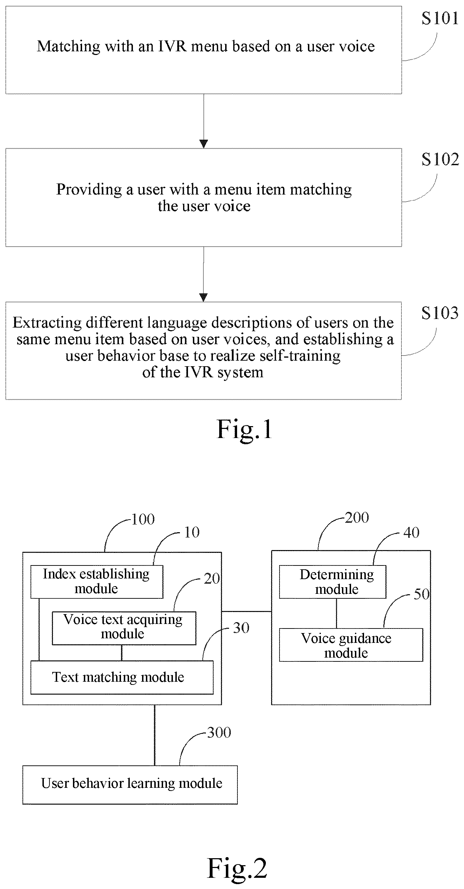

The first embodiment of the present disclosure provides a method for quickly accessing an IVR menu. As shown in Fig. 1, the method includes the following steps.

In step S101, a matching is performed in an IVR menu based on voice of a user.

In particular, step S101 includes the following process flows.

In A1, a text corresponding to the voice of the user is acquired.

Further, after prompting the user via voice a usable function of the menu item, the voice of the user is recorded and is converted into a user voice text, and then a preprocessing is performed on the user voice text. The preprocessing includes: a process of removing a stopword, and filtering out a character not encoded according to GBK and a non-word character by using a regular expression. For example, the typical non-word character is a punctuation, or a character such as &, #, and. The removal of the stopword may be performed based on a stopword dictionary. The preprocessed user voice text is the acquired text corresponding to the voice of the user.

In an embodiment, the preprocessed user voice text also needs to be split, and the split user voice text is used as the acquired text corresponding to the user voice. The splitting refers to decomposing words and expressions in the user voice text based on a characteristic or property of a certain word, the meaning of a word, or the like, so as to be better matched with a keyword in an index of the menu items.

In A2, an index of the menu items is established based on a text corresponding to the IVR menu.

In an embodiment, the IVR menu consists of individual menu items. If there are multi-level of menu items, then a certain menu item in a certain level may correspond to a plurality of menu items in a level lower than the certain level. The index of the menu items contains keywords corresponding to individual menu items for being matched with the words and expressions in the user voice text.

In A3, the text corresponding to the user voice is matched with the index of the IVR menu items, so as to find out the menu item matched with the user voice.

In an embodiment, the IVR menu in the present embodiment is an IVR menu which has been flattened.

The flattening process means: with respect to menu items in two adjacent levels in the IVR items, replacing the menu item in a current level by the menu item in a corresponding next level. For example, a certain menu item is "querying call fee", and menu items in the next level include: "querying local call fee" and "querying other call fee", then the menu item "querying call fee" is directly replaced by the next menu items "querying local call fee" and "querying other call fee". In this way, the entire menu tends to be flattened, and levels of the menu are reduced, which facilitates the user to arrive at the desired menu item quickly.

The total number of menu levels in the IVR menu may be set according to requirements, but should be less than the number of levels in the current IVR menu. The flattening process to the current IVR menu may also be performed with respect to a specific level or certain several levels selectively according to requirements. When the total number of levels of the IVR menu is reduced to be one level after the flattening process, the technical solution of the embodiments of the present disclosure is performed, such that the user can reach the desired menu item most quickly, and the optimum efficiency of the present disclosure can be achieved.

In step S102, the menu item matched with the user voice is provided to the user.

In particular, step S102 includes the following process flows.

In B1, it is determined whether a number of the menu item matched with the user is 1; if yes, business logic functionality of the menu item is provided to the user; otherwise, step B2 is performed.

In B2, a voice guidance is performed for the user based on the user voice text, so as to prompt the user to input more accurate voice and perform the matching again.

In an embodiment, the method for quickly accessing an IVR menu in the present embodiment further includes the following step.

In step S103, different language description information with respect to a same menu item by the user is extracted based on the user voice, and a user behavior library is established to realize self-training of the IVR system. In this way, a relative wide language expression range can be provided for the user at subsequent, and the menu item which the user wants to reach can be recognized more accurately.

Corresponding to the first embodiment, the second embodiment of the present disclosure introduces a device for quickly accessing an IVR menu. As shown in Fig. 2, the device includes the following constituent parts: a matching module 100 and a processing module 200.
1) The matching module 100 is configured to perform a matching in the IVR menu based on voice of a user.

In particular, the matching module 100 includes the following constituent modules: an index establishing module 10, a voice text acquiring module 20, and a text matching module 30.

The index establishing module 10 is configured to establish an index of menu items based on a text corresponding to the IVR menu. The IVR menu consists of individual menu items. If there are multi-level of menu items, then a certain menu item in a certain level may correspond to a plurality of menu items in a level lower than the certain level. The index of the menu items contains keywords corresponding to individual menu items for being matched with the words and expressions in the user voice text.

In an embodiment, the index establishing module 10 is also configured to first perform a flattening process on the IVR menu and then establish the index of the menu items based on a text corresponding to the processed IVR menu.

The flattening process means: with respect to menu items in two adjacent levels in the IVR menu, replacing the menu item in a current level by the menu item in a corresponding next level. The total number of menu levels in the IVR menu may be set according to requirements, but should be less than the number of levels in the current IVR menu. The flattening process to the current IVR menu may also be performed with respect to a specific level or certain several levels selectively according to requirements.

The voice text acquiring module 20 is configured to, after prompting the user an available function of the menu item via voice, record the voice of the user and convert it into a user voice text, and then preprocess the user voice text. The preprocessed user voice text is the acquired text corresponding to the voice of the user. The preprocessing includes: a process of removing a stopword, and filtering out a character not encoded according to GBK and a non-word character by using a regular expression. In an embodiment, the voice text acquiring module 20 is also configured to split the preprocessed user voice text, and using the split user voice text as the acquired text corresponding to the user voice. The splitting may be decomposing words and expressions in the user voice text based on a characteristic or property of a certain word, the meaning of a word, or the like, so as to be better matched with a keyword in the index of the menu items.

The text matching module 30 is configured to match a text corresponding to the user voice with the index of the IVR menu items, so as to find out the menu item matched with the user voice.
2) The processing module 200 is configured to provide the menu item matched with the user voice to the user.

In particular, the processing module 200 includes the following constituent parts: a determining module 40 and a voice guidance module 50.

The determining module 40 is configured to determine whether a number of the menu item matched with the user voice is 1; if yes, provide a business logic functionality of the menu item to the user; otherwise, call the voice guidance module 50.

The voice guidance module 50 is configured to perform a voice guidance for the user based on the user voice text, so as to prompt the user to input more accurate voice and perform the matching again. For example, the user voice is "I want to query", since there are various items for query provided by the IVR menu to the user, when voice-guiding the user based on the user voice text, "querying call fee", "querying plan flow", and "querying balances" may be broadcasted for selection by the user, and the user's selection is fed back via the natural language, and then the matching module 100 is re-called for matching.

In an embodiment, the device for quickly accessing the IVR menu in the present embodiment further includes:
3) a user behavior learning module 300 configured to extract different language description information with respect to a same menu item by the user based on the user voice, and establish a user behavior library to realize self-training of the IVR system. The user behavior learning module 300 is used for guiding the matching module 100, such that a relative wide language expression range can be provided for the user at subsequent, and the menu item which the user wants to reach can be recognized more accurately

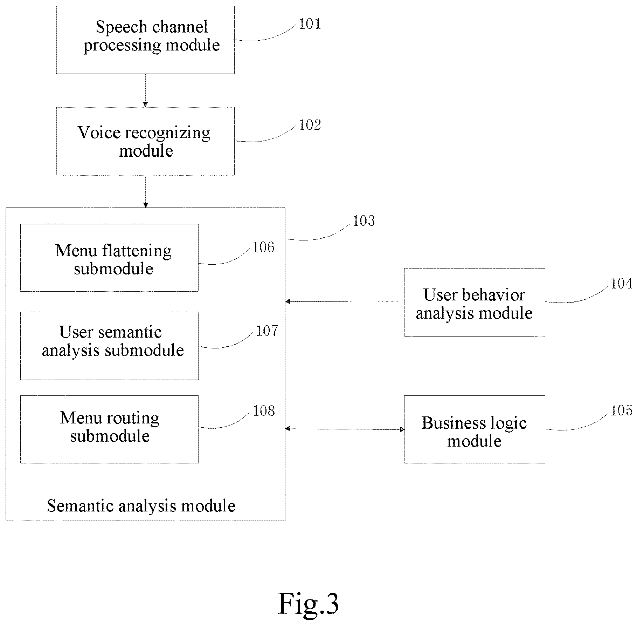

The third embodiment of the present disclosure introduces an application example of the present disclosure based on the above embodiments in combination with Figs. 3-5.

Fig. 3 is a schematic diagram of constituent structure of an IVR system according to the present embodiment. As shown in Fig. 3, the IVR system includes: a speech channel processing module 101, a voice recognizing module 102, a semantic analysis module 103, a user behavior analysis module 104 and a business logic module 105.

The speech channel processing module 101, the voice recognizing module 102, and the semantic analysis module 103 have functions similar to the matching module in the second embodiment, the user behavior analysis module 104 has functions similar to the user behavior analysis module in the second embodiment, and the business logic module 105 has functions similar to the processing module in the second embodiment.

The speech channel processing module 101 is configured to receive a voice request from a user, control and manage individual telephone channels, and transmit the recorded data to the voice recognizing module 102.

The voice recognizing module 102 is configured to recognize the voice input, i.e., convert the recorded data into text data and transfer the recognized result to the semantic analysis module.

The semantic analysis module 103 includes: a menu flattening submodule 106, a user semantic analysis submodule 107 and a menu routing submodule 108. The menu flattening submodule 106 is configured to perform a flattening process on the IVR menu to reduce levels of the menu. The user semantic analysis submodule 107 is configured to parse and identify the natural language, and acquire final information for querying the menu items. The menu routing submodule 108 is configured to make the user access the corresponding business menu item.

The user behavior analysis module 104 is configured to establish a user behavior log, extract appealing description about the menu by the user and save it into a menu information library, so as to achieve self-training of the IVR.

The business logic module 105 is configured to execute flows of business logic of the IVR, and complete individual business logic functions by interacting with the semantic analysis module 103.

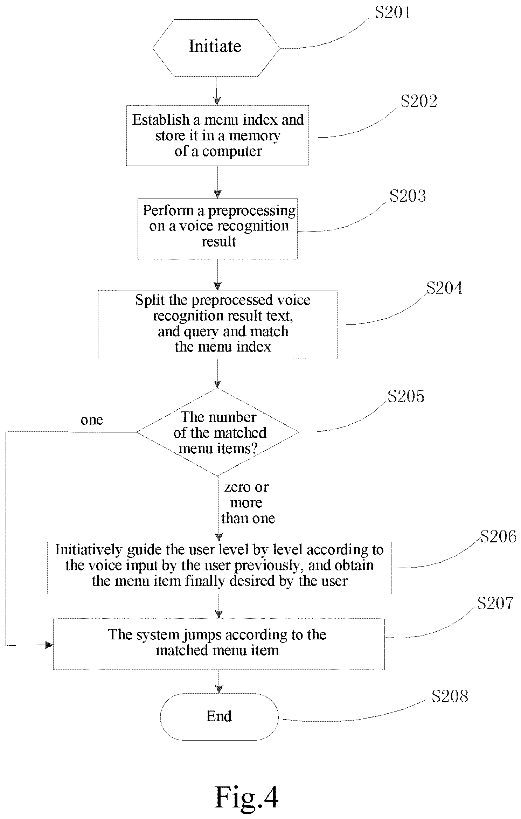

As shown in Fig. 4, the flow executed by the semantic analysis module in the present embodiment includes the following steps.

In step S201, an initiation is performed, a flattening process is performed on an IVR menu, and menu information is loaded.

In step S202, an index of the menu information is established, and is saved in a memory of a computer.

In step S203, a preprocessing is performed on a voice recognition result. The preprocessing mainly includes removing a stopword and filtering out non-GBK character and non-word character in the voice recognition result text.

In step S204, the preprocessed voice recognition result text is split, and querying and matching are performed on the index of the menu.

In step S205, a number of the matched menu items in the querying result of the menu index is determined; if the number is 0 or more than 1, the procedure goes to step S206; and if the number is 1, the procedure directly enters step S207.

In step 206, the user is initiatively guided level by level according to the voice input by the user previously, and the menu item finally desired by the user is obtained.

In step 207, the IVR system jumps according to the matched menu item.

In step 208, the analysis ends.

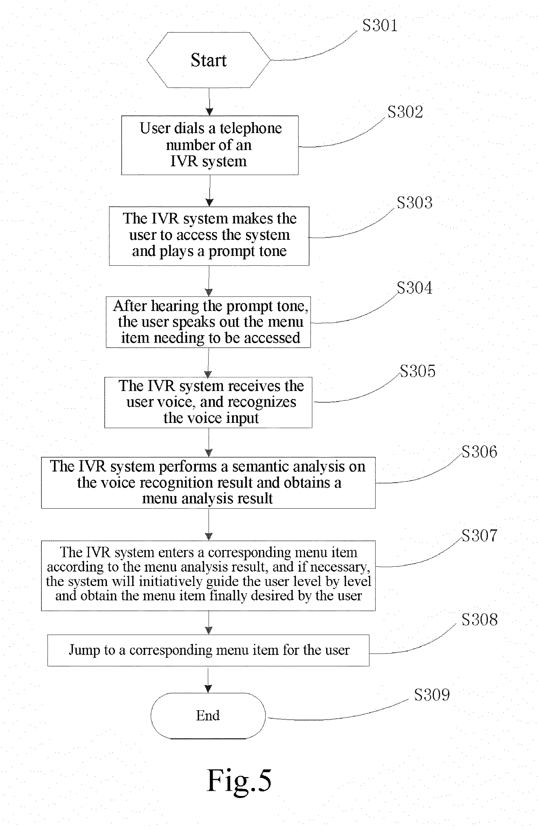

As shown in Fig. 5, the flow of quickly accessing the IVR menu in the present embodiment includes the following steps.

In step S301, the flow starts.

In step S302, a user dials a telephone number of the IVR system.

In step S303, the IVR system makes the user to access the system and a prompt tone is played.

In step S304, after hearing the prompt tone, the user speaks out the menu item needing to be accessed.

In step S305, the IVR system receives the user voice, and recognizes the voice input.

In step S306, the IVR system performs a semantic analysis on the voice recognition result and obtains a menu analysis result.

In step S307, the IVR system enters a corresponding menu item according to the menu analysis result, and if necessary, the system will initiatively guide the user level by level and obtain the menu item finally desired by the user.

In step S308, a corresponding menu item is jumped to for the user.

In step S309, the flow ends.

The method and device for quickly accessing the IVR menu in the present disclosure enable the user to quickly arrive at the desired menu item in the IVR menu via direct interaction in natural language, and reduce the barrier in using the IVR system by the user, which are particularly embodied in the following four aspects of advantages.
1) The IVR menu is accessed by direct interaction in natural language without using keys, which saves the waiting time of the user, and improves the usage experience of the IVR.
2) The IVR menu is flattened, the levels of the menu are reduced, and the user can be initiatively guided level by level to enable the user to quickly access the desired menu.
3) The self-training of the IVR can be achieved according to the user's behaviors, so as to improve the intelligence of the IVR.
4) The condition in which the interaction time is prolonged due to a misoperation on the keys by the user is avoided.

Through the explanations of particular implementation manners, the technical means adopted by the present disclosure for achieving predefined purposes and the effects may be understood more deeply and specifically. However, the drawings are only provided for reference and explanations, instead of limiting the present disclosure.

### INDUSTRIAL APPLICABILITY

The above technical solutions provided by the present disclosure may be applied in the procedure of quickly accessing an Interactive Voice Response IVR menu. By using the technical solution of matching in the IVR menu based on the user's voice and providing the menu item matched with the user's voice to the user, the user is capable of quickly reaching the desired menu item in the IVR menu by direct interaction in natural language, the waiting time of the user is saved and the usage experience of the IVR is improved. Preferably, the flattening process is performed on the IVR menu to reduce the levels of the menu. When the voice inputted by the user is not accurate, the user can be actively guided level by level according to the voice inputted by the user previously, such that the user can quickly go into the desired menu.

## Claims

**1.** A method for quickly accessing an Interactive Voice Response IVR menu, comprising:
performing a matching in the IVR menu based on voice of a user, and providing a menu item matched with the voice of the user to the user.

**2.** The method for quickly accessing an IVR menu according to claim 1, wherein the performing the matching in the IVR menu based on the voice of the user comprises:
establishing an index of menu items based on a text corresponding to the IVR menu; and
matching a text corresponding to the voice of the user with the index of the menu items so as to find out the menu item matched with the voice of the user.

**3.** The method for quickly accessing an IVR menu according to claim 1 or 2, wherein the IVR menu is an IVR menu which has undergone a flattening process, and
the flattening process comprises: with respect to menu items in two adjacent levels in the IVR menu, replacing the menu item in a current level by the menu item in a corresponding next level.

**4.** The method for quickly accessing an IVR menu according to claim 2, wherein a procedure of acquiring the text corresponding to the voice of the user comprises:
after prompting the user an available function of the menu item via voice, recording the voice of the user and converting it into a user voice text, and then preprocessing the user voice text, the preprocessing comprising: a process of removing a stopword, and filtering out a character not encoded according to Chinese Internal Code Specification GBK and a non-word character.

**5.** The method for quickly accessing an IVR menu according to claim 4, wherein the procedure of acquiring the text corresponding to the voice of the user further comprises:
splitting the preprocessed user voice text.

**6.** The method for quickly accessing an IVR menu according to claim 2, wherein the providing the menu item matched with the voice of the user to the user comprises:
A1: determining whether a number of the menu item matched with the voice of the user is 1; if yes, providing a business logic functionality of the menu item to the user; otherwise, performing step A2; and
A2: performing a voice guidance for the user based on the user voice text, so as to prompt the user to input more accurate voice and perform the matching again.

**7.** A device for quickly accessing an Interactive Voice Response IVR menu, comprising:
a matching module configured to perform a matching in the IVR menu based on voice of a user; and
a processing module configured to provide a menu item matched with the voice of the user to the user.

**8.** The device for quickly accessing an IVR menu according to claim 7, wherein the matching module comprises:
an index establishing module configured to establish an index of menu items based on a text corresponding to the IVR menu; and
a text matching module configured to match a text corresponding to the voice of the user with the index of the menu items so as to find out the menu item matched with the voice of the user.

**9.** The device for quickly accessing an IVR menu according to claim 7 or 8, wherein the matching module comprises:
a menu processing module configured to perform a flattening process on the IVR menu and then provide it to the matching module, and
the flattening process comprises: with respect to menu items in two adjacent levels in the IVR menu, replacing the menu item in a current level by the menu item in a corresponding next level.

**11.** An Interactive Voice Response IVR system, comprising:
a recording device configured to record voice of a user and convert it into a user voice text; and
a processor configured to establish an index of menu items based on a text corresponding to an IVR menu, match the user voice text with the index of the menu items so as to find out the menu item matched with the voice of the user, and provide business logic functionality of the menu item to the user.
